**Europäisches Patentamt**

**European Patent Office**

**Office européen des brevets**

(11) Publication number : **0 504 307 B1**

# EUROPEAN PATENT SPECIFICATION

(45) Date of publication of patent specification :
**19.01.94 Bulletin 94/03**

(51) Int. Cl.⁵ : **A61K 39/395, C12P 21/08, C12N 5/10**

(21) Application number : **91902317.6**

(22) Date of filing : **30.11.90**

(86) International application number :
**PCT/US90/06872**

(87) International publication number :
**WO 91/07986 13.06.91 Gazette 91/13**

(54) **METHOD OF TREATING SEPTIC SHOCK.**

(30) Priority : **04.12.89 US 445192**

(43) Date of publication of application :
**23.09.92 Bulletin 92/39**

(45) Publication of the grant of the patent :
**19.01.94 Bulletin 94/03**

(84) Designated Contracting States :
**AT BE CH DE DK ES FR GB GR IT LI LU NL SE**

(56) References cited :
**EP-A- 0 312 996**
**EP-A- 0 326 120**
**EP-A- 0 327 283**
**EP-A- 0 399 429**
**HYBRIDOMA, vol. 8, no. 5, 1989, Mary Ann Liebert Inc. Plc., New York, NY (US); D. NOVICK et al., pp. 561-567**

(73) Proprietor : **SCHERING CORPORATION**
**2000 Galloping Hill Road**
**Kenilworth New Jersey 07033 (US)**
Proprietor : **STARNES, H. Fletcher**
**2210 High Street**
**Palo Alto, CA 94301 (US)**

(72) Inventor : **STARNES, H., Fletcher**
**2210 High Street**
**Palo Alto, CA 94301 (US)**
Inventor : **ABRAMS, John, S.**
**1817 Oak Knoll Drive**
**Belmont, CA 94002 (US)**

(74) Representative : **Ritter, Stephen David et al**
**Mathys & Squire 10 Fleet Street**
**London EC4Y 1AY (GB)**

## Description

FIELD OF THE INVENTION

The invention relates to a method of treating septic shock by administering antagonists to interleukin-6 (IL-6).

BACKGROUND

Severe infections, particularly with gram negative bacteria, can result in profound physiological and metabolic alterations including fever, hypotension, metabolic acidosis, widespread organ disfunction and ultimately death. Such alterations are frequently referred to as septic shock. The host-derived protein, tumor necrosis factor (TNF), has recently been demonstrated to induce many of the deleterious effects of gram negative septicemia, and passive immunization with antisera to TNF can prevent the lethal effects of gram negative bacteremia or endotoxemia: e.g. Tracey et al., Science, Vol. 234, pg. 470 (1986); Beutler et al., Nature, Vol. 320, pg. 584 (1986); and Tracey et al., Nature, Vol. 330, pg. 662 (1987). High serum levels of TNF have been observed in several infectious diseases including meningococcal meningitis, malaria, and leishmaniasis, and patients with high circulating levels of TNF have increased organ dysfunction along with higher mortality: e.g. Waage et al., Lancet, pg. 355 (1987); Girardin et al., New Engl. J. Med., Vol. 319, pg. 397 (1988); Scuderi et al., Lancet, pg. 1364 (1988); and Kern et al., Am. J. Med., Vol. 87, pg. 139 (1989).

It has been reported that administration of TNF either to animals or to human subjects resulted in detectable IL-6 serum levels two to six hours later: McIntosh et al., J. Immunol., Vol. 143, pg. 162 (1989); Jablons et al., J. Immunol., Vol. 142, pg. 1542 (1989); and Brouckaert et al., J. Exp. Med., Vol. 169, pg. 2257 (1989). IL-6, also known as B cell stimulating factor-2, interferon-$\beta_2$, and hepatocyte stimulating factor, has been identified as a T cell-derived glycoprotein that causes B cell maturation, e.g. Kishimoto, Blood, Vol. 74, pg. 1 (1989). More recently, IL-6 has been demonstrated to possess pleiotropic biological activities, including induction of acute phase proteins in hepatocytes and actions on hematopoietic progenitor cell and T cells: e.g. Geiger et al., Eur. J. Immunol., Vol. 18, pg. 717 (1988); Marinjovic et al., J. Immunol., Vol. 142, pg. 808 (1989); Morrone et al., J.Biol. Chem., Vol. 263, pg. 12554 (1988); and Perlmutter et al., J. Clin. Invest., Vol. 84, pg. 138 (1989).

Presently, septic conditions, such as bacteremia and the like, are treated with antimicrobial compounds. However, when such conditions are associated with shock, there are no effective adjunctive measures in the therapy for ameliorating the shock syndrome: e.g. Young, pgs. 468-470, in Mandell et al., eds., Principles and Practice of Infectious Diseases, 2nd Ed. (John Wiley &Sons, New York, 1985). In view of this, the availability of techniques for treating shock associated with sepsis could have important clinical utility.

SUMMARY OF THE INVENTION

The invention provides a method for the use of an antagonist against human IL-6 for the preparation of a pharmaceutical composition useful in the treatment of septic shock. Preferably, the antagonists to IL-6 are monoclonal antibodies, or binding compositions derived therefrom by standard techniques.

BRIEF DESCRIPTION OF THE DRAWINGS

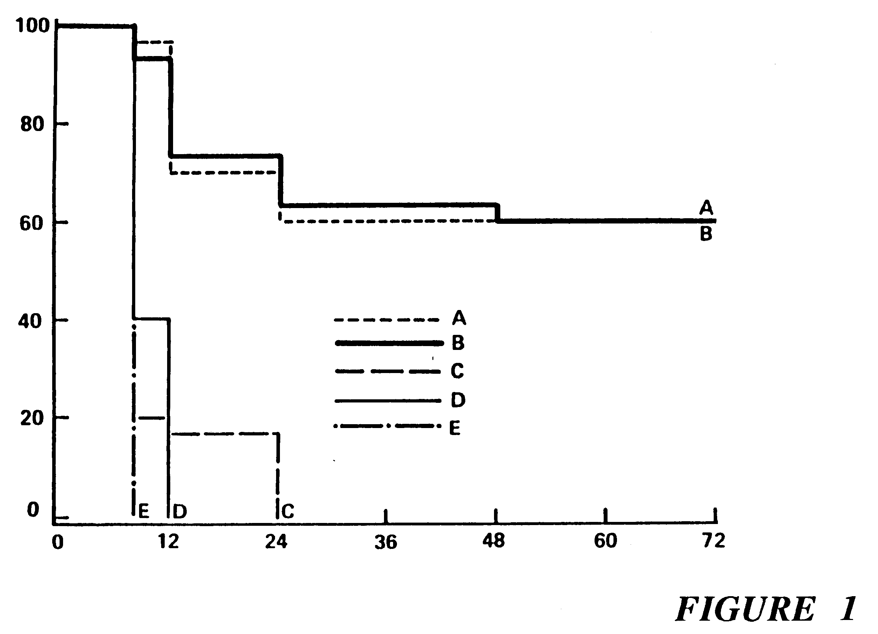

Figure 1 illustrates survival data of male BALB/c mice which were injected intraperitoneally with live E. coli and treated with various monoclonal antibodies.

DETAILED DESCRIPTION OF THE INVENTION

The invention is based on the discovery that increased production of IL-6 is a link in a causal chain of physiological events leading to shock in massive microbial infections. such as seoticemia. The method of the invention comprises the use of an antagonist against human IL-6 for the preparation of a pharmaceutical composition useful in the treatment of septic shock.

Preferably, antagonists used in the invention are derived from antibodies specific for human IL-6. More preferably, the antagonist used in the invention comprise fragments or binding compositions specific for IL-6. Antibodies comprise an assembly of polypeptide chains linked together by disulfide bridges. Two major polypeptide chains, referred to as the light chain and the heavy chain, make up all major structural classes (isotypes) of antibody. Both heavy chains and light chains are further divided into subregions referred to as variable regions and constant regions. Heavy chains comprise a single variable region and three different constant regions, and light chains comprise a single variable region (different from that of the heavy chain) and a single constant region (different from those of the heavy chain). The variable regions of the heavy chain and light chain are responsible for the binding specificity of the antibody.

As used herein, the term "heavy-chain variable region" means a polypeptide (1) which is from 110 to 125 amino acids in length, and (2) whose amino acid sequence corresponds to that of a heavy chain of a monoclonal antibody of the invention, starting from the heavy chain's N-terminal amino acid. Likewise, the term "light-chain variable region" means a polypeptide (1) which is from 95 to 115 amino acids in length, and (2) whose amino acid sequence corresponds to that of a light chain of a monoclonal antibody of the invention, starting from the light chain's N-terminal amino acid. As used herein the term "monoclonal antibody" refers to homogeneous populations of immunoglobulins which are capable of specifically binding to human IL-6.

As used herein the term "binding composition" means a composition comprising two polypeptide chains, (1) which, when operationally associated, assume a conformation having high binding affinity for human IL-6, and (2) which are derived from a hybridoma producing monoclonal antibodies specific for human IL-6. The term "operationally associated" is meant to indicate that the two polypeptide chains can be positioned relative to one another for binding by a variety of means, for example, association in a native antibody fragment, such as Fab or Fv, or by way of genetically engineered cysteine-containing peptide linkers at the carboxyl termini. Normally, the two polypeptide chains correspond to the light-chain variable region and heavy-chain variable region of a monoclonal antibody specific for human IL-6. Preferably, antagonists of the invention are derived from monoclonal antibodies specific for human IL-6. Monoclonal antibodies capable of blocking, or neutralizing, IL-6 are selected by their ability to inhibit IL-6-induced effects in standard IL-6 bioassays: e.g. as described in Sehgal et al., Science, Vol. 235, pgs. 731-732 (1987); Billiau, Immunol. Today, Vol. 8, pgs. 84-87 (1987); and Wong et al., Immunol. Today, Vol. 9, pgs. 137-139 (1988). Preferably, inhibition of the following biological activities is used to screen for monoclonal antibodies capable of neutralizing IL-6: (i) antiviral activity, (ii) enhancement of immunoglobulin secretion, and (iii) stimulation of growth of certain EBV-transformed cell lines. Inhibition of antiviral activity can be measured in standard cytopathic effect inhibition assays, such as those described by Armstrong, Meth. Enzymol., Vol. 78, pgs. 381-387 (1981), and Familletti et al., Meth. Enzymol., vol. 78, pgs. 387-399 (1981), both of which are incorporated by reference. Neutralizing antibodies are selected on the basis of their ability to counteract, or negate, inhibition of cytopathic effects of a standard concentration of IL-6. Neutralizing antibodies can also be selected by their ability to prevent IL-6-stimulated growth of certain plasmacytoma cell lines, such as MOPC-315, e.g. Chiu et al., Proc. Natl. Acad. Sci. USA, Vol. 85, pgs. 7099-7103 (1988). MOPC-315 is available from the American Type Culture Collection under accession number TIB 23. Another means for selecting neutralizing antibodies is the inhibition of IL-6-stimulated immunoglobulin secretion of EBV-transformed cell lines, such as CESS and SKW6.4 (available from the ATCC under accession numbers TIB 190 and TIB 215, respectively), e.g. Hirano et al., Proc. Natl. Acad. Sci. USA, Vol. 82, pgs. 5490-5494 (1985). Antibody secretion is conveniently measured by an isotype-specific ELISA using commercially available isotype-specific monoclonal antibodies. For example, (4-6) x $10^3$ CESS or SKW 6.4 cells are cultured in 200 µl of culture medium, e.g. RPMI 1640, with about 3-30 pM of IL-6. After about 3 days the supernatants are assayed for IgG concentration (CESS) or for IgM concentration (SKW 6.4).

Hybridomas are produced by well known techniques. Usually, the process involves the fusion of an immortalizing cell line with a B-lymphocyte which produces the desired antibody. Alternatively, non-fusion techniques for generating immortal antibody-producing cell lines are possible, and come within the purview of the present invention, e.g. virally induced transformation: Casali et al., "Human Monoclonals from Antigen-Specific Selection of B Lymphocytes and Transformation by EBV," Science, Vol. 234, pgs. 476-479 (1986). Immortalizing cell lines are usually transformed mammalian cells, particularly myeloma cells of rodent, bovine, and human origin. Most frequently, rat or mouse myeloma cell lines are employed as a matter of convenience and availability. Techniques for obtaining the appropriate lymphocytes from mammals injected with the target antigen are well known. Generally, either peripheral blood lymphocytes (PBLs) are used if cells of human origin are desired, or spleen cells or lymph node cells are used if non-human mammalian sources are desired. A host mammal is injected with repeated dosages of the purified antigen, and the mammal is permitted to generate the desired antibody-producing cells before these are harvested for fusion with the immortalizing cell line. Techniques for fusion are also well known in the art and, in general, involve mixing the cells with a fusing agent, such as polyethylene glycol. Hybridomas are selected by standard procedures, such as HAT selection. From among these hybridomas, those secreting the desired antibody, i.e. one specific for human IL-6, are selected by assaying their culture medium by standard immunoassays, such as Western blotting, ELISA, RIA, IL-6 neutralizing capability, or the like. Antibodies are recovered from the medium using standard protein purification techniques, e.g. Tijssen, Practice and Theory of Enzyme Immunoassays (Elsevier, Amsterdam, 1985). Many references are available for guidance in applying any of the above techniques: e.g. Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and the like. Hybridomas producing monoclonal antibodies specific for human IL-6 are then subjected to a second screen using the IL-6 assays described above to select ones capable of blocking, or neutralizing, the biological activity of IL-6.

The use and generation of fragments of antibodies is also well known, e.g. Fab fragments: Tijssen, Practice and Theory of Enzyme Immunoassays (Elsevier, Amsterdam, 1985); and Fv fragments: Hochman et al. Biochemistry, Vol. 12, pgs. 1130-1135 (1973), Sharon et al., Biochemistry, Vol. 15, pgs. 1591-1594 (1976), and Ehrlich et al., U.S. Patent 4,355,023; and antibody half molecules: Auditore-Hargreaves, U.S. Patent 4,470,925.

Hybridomas and monoclonal antibodies are produced against either glycosylated or unglycosylated versions of recombinantly produced mature human IL-6. Generally, unglycosylated versions of human IL-6 are produced in E. coli, and glycosylated versions are produced in mammalian cell hosts, e.g. CV1 or COS monkey cells, mouse L cells, or the like. Recombinantly produced mature human IL-6 is produced by introducing an expression vector into a host cell using standard protocols: e.g. Maniatis et al., Molecular Cloning: A Laboratory Manual (Cold Spring Harbor Laboratory, New York, 1982); Okayama and Berg, Mol. Cell. Biol., Vol. 2, pgs. 161-170 (1982), and Vol. 3, pgs. 280-289 (1983); Takebe et al., Mol. Cell. Biol., Vol. 8, pgs. 466-472 (1988); U.S. patent 4,599,308; U.S. patent 4,675,285; Kaufman et al., Mol. Cell. Biol., Vol. 2, pgs. 1304-1319 (1982); or the like. Construction of bacterial or mammalian expression vectors is well known in the art, once the nucleotide sequence encoding a desired protein is known or otherwise available: e.g., DeBoer in U.S. Patent 4,551,433 discloses promoters for use in bacterial expression vectors; Goeddel et al., in U.S. Patent 4,601,980, and Riggs, in U.S. Patent 4,431,739, disclose the production of mammalian proteins by E.coli expression systems; and Riggs (cited above), Ferretti et al., Proc. Natl. Acad. Sci. USA, Vol. 83, pgs. 599-603 (1986), Sproat et al., Nucleic Acids Research, Vol.13, pgs. 2959-2977 (1985), and Mullenbach et al., J. Biol. Chem., Vol. 261, pgs. 719-722 (1986) disclose how to construct synthetic genes for expression in bacteria. Accordingly, these references are incorporated by reference. The amino acid sequence of mature human IL-6 is disclosed by Hirano et al., Nature, Vol. 324, pgs. 73-76 (1986) and Zilberstein et al., EMBO J., Vol. 5, pgs. 2529-2537 (1986); synthetic genes encoding human IL-6 are available commercially from Beckman Instruments (Fullerton, CA); and purified human IL-6 is commercially available from Genzyme Corp. (Boston, MA). Many bacterial expression vectors and hosts are available commercially and through the ATCC.

Antibodies and antibody fragments characteristic of hybridomas can also be produced by recombinant means by extracting messenger RNA, constructing a cDNA library, and selecting clones which encode segments of the antibody molecule: e.g. Wall et al., Nucleic Acids Research, Vol. 5, pgs. 3113-3128 (1978); Zakut et al., Nucleic Acids Research, Vol. 8, pgs. 3591-3601(1980); Cabilly et al., Proc. Natl. Acad. Sci. USA, Vol. 81, pgs. 3273-3277 (1984); Boss et al., Nucleic Acids Research, Vol. 12, pgs. 3791-3806 (1984); Amster et al., Nucleic Acids Research, Vol. 8, pgs. 2055-2065 (1980); Moore et al., U.S. Patent 4,642,334; and Skerra et al., Science, Vol. 240, pgs. 1038-1041(1988). In particular, such techniques can be used to produce interspecific monoclonal antibodies, wherein the binding region of one species is combined with non-binding region of the antibody of another species to reduce immunogenicity, e.g. Liu et al., Proc. Natl. Acad. Sci., Vol. 84, pgs. 3439-3443 (1987).

Antagonists used in the invention are administered as a pharmaceutical composition. Such compositions contain a therapeutic amount of such an antagonist, e.g. at least one of the monoclonal antibodies, or fragments thereof, in a pharmaceutical carrier. A pharmaceutical carrier can be any compatible, non-toxic substance suitable for delivering the compositions of the invention to a patient. Sterile water, alcohol, fats, waxes, and inert solids may be included in a carrier. Pharmaceutically accepted adjuvants (buffering agents, dispersing agents) may also be incorporated into the pharmaceutical composition. Generally, compositions useful for parenteral administration of such drugs are well known, e.g. Remington's Pharmaceutical Science, 15th Ed. (Mack Publishing Company, Easton, PA 1980). Alternatively, compositions of the invention may be introduced into a patient's body by implantable or injectable drug delivery system: e.g. Urquhart et al., Ann. Rev. Pharmacol. Toxicol., Vol. 24, pgs. 199-236 (1984); Lewis, ed. Controlled Release of Pesticides and Pharmaceuticals (Plenum Press, New York, 1981); U.S. patent 3,773,919; U.S. patent 3,270,960; and the like.

Antagonists used in the invention that are derived from antibodies are normally administered parentally, preferably intravenously. Since such protein or peptide antagonists may be immunogenic, they are preferably administered slowly, either by a conventional IV administration set or from a subcutaneous depot, e.g. as taught by Tomasi et al., U.S. patent 4,732,863. When administered parenterally the antibodies or fragments will be formulated in a unit dosage injectable form (e.g., solution, suspension or emulsion) in association with a pharmaceutically acceptable parenteral vehicle. Such vehicles are inherently nontoxic and nontherapeutic. Examples of such vehicles are normal saline, Ringer's solution, dextrose solution, and Hank's solution. Nonaqueous vehicles such as fixed oils and ethyl oleate may also be used. A preferred vehicle is 5% dextrose/saline. The vehicle may contain minor amounts of additives such as substances that enhance isotonicity and chemical stability, e.g., buffers and preservatives. The antibody is preferably formulated in purified form substantially free of aggregates, other proteins, endotoxins, and the like, at concentrations of about 5 to 30 mg/ml, preferably 10 to 20 mg/ml. Preferably, the endotoxin levels are less than 2.5 EU/ml.

Selecting an administration regimen for an antagonist depends on several factors, including the serum turnover rate of the antagonist, the serum level of IL-6 associated with the septecemia, the immunogenicity of the antagonist, the accessibility of the target IL-6 (e.g. if non-serum IL-6 is to be blocked), the affinity of IL-6 to its receptor(s) relative to that of IL-6 to the antagonist, and the like. Preferably, an administration regimen maximizes the amount of antagonist delivered to the patient consistent with an acceptable level of side effects. Accordingly, the amount of antagonist delivered depends in part on the particular antagonist and the severity of the condition being treated. Guidance in selecting appropriate doses is found in the literature on therapeutic uses of antibodies: e.g. Bach et al., chapter 22, in Ferrone et al., eds., Handbook of Monoclonal Antibodies (Noges Publications, Park Ridge, NJ, 1985); and Russell, pgs. 303-357, and Smith et al., pgs. 365-389, in Haber et al., eds., Antibodies in Human Diagnosis and Therapy (Raven Press, New York, 1977). Preferably, whenever the antagonist comprises monoclonal antibodies or Fab-sized fragments thereof (including binding compositions), the dose is in the range of about 1-20 mg/kg per day. More preferably the dose is in the range of about 1-10 mg/kg per day.

## EXAMPLES

The following examples serve to illustrate aspects of the present invention. The selected vectors, hosts, fusion partners, concentration of reagents, temperatures, and the values of other variables are only to exemplify the invention and are not to be considered limitations thereof.

Example I. Production of IL-6-Neutralizing Monoclonal Antibody

A male Lewis rat was immunized with semi-purified preparations of COS7-cell expressed mouse IL-6 (this expression system being described by Lee et al., Annals N.Y. Acad. Sci., Vol. 557, pgs. 215-229 (1989) and by Chui et al., Proc. Natl. Acad. Sci. USA, Vol. 85, 7099-7103 (1988)). The rat was first immunized with approximately 50 μg of mouse IL-6 in Freund's Complete Adjuvant, and boosted twice with the same amount of material in Freund's Incomplete Adjuvant. Test bleeds were taken. The animal was given a final boost of 25 μg in phosphate-buffered saline, and four days later the spleen was obtained for fusion.

Approximately $3 \times 10^8$ rat splenocytes were fused with an equal number of P3X63-AG8.653 mouse myeloma cells (available from the ATCC under accession number CRL 1580). 3840 microtiter plate wells were seeded at $5.7 \times 10^4$ parental myeloma cells per well. Half of these wells were initially cultured with approximately 22 units/well of recombinant human IL-6.

Units are defined in relation to the growth of the cell line NFS-60 in response to human IL-6, described by Chiu et al. (cited above). Another 576 wells were seeded at $1.9 \times 10^5$ parents per well. Standard protocols for the fusion and subsequent culturing of hybrids were followed, and were substantially the same as those described by Chretien et al., J. Immunol. Meth., Vol. 117, pgs. 67-81 (1989). Twelve days after fusion, supernatants were harvested and screened (1) by indirect ELISA on PVC plates coated with COS7-produced mouse IL-6, and (2) by their ability to inhibit growth of NFS-60 cells, which are IL-6 dependent. The above screening protocol was used because of the hybridoma growth factor activity attributed to IL-6. It was reasoned that neutralizing anti-IL-6 antibodies initially produced by these hybridomas might prevent their growth by depleting the IL-6 in the culture medium. Thus, some wells were seeded with human IL-6 (hIL-6) which is active on mouse cells. In every case about the same fraction of neutralizing antibodies was produced, as shown in the Table below.

## TABLE I

| Initial seeding condition | Number of wells containing neutralizing anti-IL-6 | Number of wells seeded | Percent of seeded wells with neutralizing Mabs |
|---|---|---|---|
| No hIL-6; $5.7 \times 10^4$ cells/well | 7 | 1920 | 0.36% |
| 22 U/well hIL-6; $5.7 \times 10^4$ cells/well | 8 | 1920 | 0.41% |
| No hIL-6; $1.9 \times 10^5$ cells/well | 6 | 576 | 1.0% |

Monoclonal antibody from a hybridoma designated MP5-20F3 was used in the experiments of Example II.

Example II. Prophylactic effect of neutralizing anti-IL-6 Mab on mice injected with a lethal dose of E. coli

Male BALB/c mice (19-21 g) were randomly assigned to five different treatment groups A - E, and experiments involving the groups were set up on five different days. In Group A (30 mice), each mouse was given by intraperitoneal injection 0.75 to 1.00 mg of monoclonal antibody (Mab) against recombinant mouse IL-6 (anti-recombinant mouse IL-6 monoclonal antibody). In Group B (30 mice), each mouse was given by intraperitoneal injection 0.1 to 1.0 mg rabbit anti-recombinant mouse $TNF\alpha$ IgG. In Group C (30 mice), each mouse was given by intraperitoneal injection 0.5 to 1.0 mg isotype control Mab GL113. In Group D (5 mice), each mouse was given by intraperitoneal injection an isotype control Mab with a 2-fold higher level of endotoxin than that of the anti-IL-6 Mab. Finally, in Group E (6 mice), each mouse was given by intraperitoneal injection a 100-fold lower dose of anti-recombinant mouse IL-6 than given to mice in Group A. For each group, two hours after the initial injection, each mouse was given by intraperitoneal injection $1.2 \times 10^8$ live E.coli (obtained from the ATCC under accession number 25922) in 0.2 to 0.3 ml of saline. The E. coli were cultured on trypticase soy agar plates with 5% sheep blood and were counted by spectrophotometric determination. The effects of the various experimental regimens on the survival of the mice are shown in Figure 1, where the y-axis shows the percent of surviving mice and the x-axis shows the time in hours after the mice were injected with E.coli. Only the groups of mice receiving a high dose of anti IL-6 antibody or rabbit anti-recombinant mouse $TNF\alpha$ IgG showed any survival more than 24 hours after injection with E.coli; these two groups of mice showed a significant survival rate even 72 hours after injection with E. coli.

## Claims

1. The use of an antagonist against human IL-6 for the preparation of a pharmaceutical composition useful in the treatment of septic shock.

2. The use claimed in Claim 1 wherein said antagonist to human IL-6 is a monoclonal antibody capable of blocking the biological activity of human IL-6, a fragment of a monoclonal antibody capable of blocking the biological activity of human IL-6, or a binding composition comprising the heavy-chain variable region and light-chain variable region of a monoclonal antibody capable of blocking the biological activity of human IL-6.

3. The use claimed in Claim 2 wherein said fragment of said monoclonal antibody is an Fab fragment.

4. The use claimed in Claim 2 wherein said monoclonal antibody is produced by a human-human hybridoma.

5. The use claimed in Claim 4 wherein said fragment of said monoclonal antibody is an Fab fragment.

6. The use claimed in any preceding claim wherein said pharmaceutical composition is suitable for intravenous delivery of an amount of said antagonist in the range of about 1-20 mg/kg body weight of said individual per day.


**Patentansprüche**

1. Verwendung eines Antagonisten gegen Human-IL-6 zur Herstellung einer pharmazeutischen Zusammensetzung, die bei der Behandlung eines septischen Schocks von Nutzen ist.

2. Verwendung nach Anspruch 1, worin der Antagonist gegen Human-IL-6 ein monoklonaler Antikörper, der die biologische Aktivität von Human-IL-6 zu blockieren vermag, ein Fragment eines monoklonalen Antikörpers, der die biologische Aktivität von Human-IL-6 zu blockieren vermag, oder eine bindende Zusammensetzung, die einen variablen Bereich der schweren Kette und einen variablen Bereich der leichten Kette eines monoklonalen Antikörpers enthält, der die biologische Aktivität von Human-IL-6 zu blockieren vermag, ist.

3. Verwendung nach Anspruch 2, worin das Fragment des monoklonalen Antikörpers ein Fab-Fragment ist.

4. Verwendung nach Anspruch 2, worin der monoklonale Antikörper durch ein Human-Human-Hybridom erzeugt wird.

5. Verwendung nach Anspruch 4, worin das Fragment des monoklonalen Antikörpers ein Fab-Fragment ist.

6. Verwendung nach irgendeinem vorhergehenden Anspruch, worin die pharmazeutische Zusammensetzung zur intravenösen Verabreichung in einer Menge des Antagonisten im Bereich von etwa 1 bis 20 mg/kg Körpergewicht des Individuums pro Tag geeignet ist.


**Revendications**

1. Utilisation d'un antagoniste de l'IL-6 humain pour préparer une composition pharmaceutique pouvant être utilisée dans le traitement du choc septique.

2. Utilisation selon la revendication 1, dans laquelle l'antagoniste de l'IL-6 humain est un anticorps monoclonal pouvant bloquer l'activité biologique de l'IL-6 humain, un fragment d'un anticorps monoclonal pouvant bloquer l'activité biologique de l'IL-6 humain, ou une composition de liaison comprenant la région variable à chaine lourde et la région variable à chaîne légère d'un anticorps monoclonal pouvant bloquer l'activité biologique de l'IL-6 humain.

3. Utilisation selon la revendication 2, dans laquelle le fragment d'anticorps monoclonal est un fragment Fab.

4. Utilisation selon la revendication 2, dans laquelle l'anticorps monoclonal est produit par un hybridome humain-humain.

5. Utilisation selon la revendication 4, dans laquelle ledit fragment d'anticorps monoclonal est un fragment Fab.

6. Utilisation selon l'une quelconque des revendications précédentes, dans laquelle la composition pharmaceutique convient à l'administration intraveineuse d'une quantité de l'antagoniste comprise dans l'intervalle d'environ 1 à 20 mg/kg de poids corporel du patient par jour.

EP 0 504 307 B1

*FIGURE 1*